# EUROPEAN PATENT APPLICATION

(11) **EP 1 346 739 A1**
(43) Date of publication of application: **24.09.2003**
(21) Application number: 03006193.1
(22) Date of filing: 19.03.2003
(51) Int. Cl.: A61M 5/32

(54) **Pivoting safety shield for needle devices**

(30) Priority: 20.03.2002 US 366368 P
(71) Applicant: Becton, Dickinson and Company, Franklin Lakes, New Jersey 07417-1880 (US)
(72) Inventor: Wilkinson, Bradley M., New Jersey 07508 (US)
(74) Representative: Müller-Boré & Partner Patentanwälte

(57) **Abstract**

A safety assembly includes a holder (10) with a proximal end, a distal end and a tubular sidewall extending between the ends. The distal end (16) is configured to engage a needle assembly (28). The proximal end (14) is configured to slidably receive a fluid collection tube. A shield (60) is hingedly mounted to the distal end (16) of the holder and can rotate from a first position where the shield lies adjacent the holder to a second position where the needle is shielded. A spring (76) is disposed for biasing the shield to the second position. A latch (82) is disposed on the holder for movement between a locking position where the latch holds the shield in the first position and a release position where the latch permits the shield to be propelled to the second position.

## Description

### FIELD OF THE INVENTION

The present invention relates to a shield for a needle and more particularly to a safety shield that may be used in conjunction with a needle assembly and a needle holder.

### BACKGROUND OF THE INVENTION

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies.

Therefore, there exists a need for a safety shield assembly: (i) that is manufactured easily; (ii) that is applicable to many devices; (iii) that is simple to use with one hand; (iv) that can be disposed of safely; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after. the needle is withdrawn from the patient's vein.

### SUMMARY OF THE INVENTION

The present invention is a safety shield assembly that comprises: a shield; means for connecting the shield to a fluid handling device that contains a piercing element, such as needle; means for securely covering and/or containing the needle within the shield and means for securely locking the shield in a final non-retractable closed position over the needle.

Preferably, the shield comprises a rearward end, a forward end, a slot or longitudinal opening for housing the used needle in the forward end, means for securing the needle in the slot, means for guiding the needle into the slot, means for connecting the shield and the fluid handling device, means for guiding the user's fingers to move the shield into various positions, and means for retaining the shield securely over the used needle.

The shield may be connected to the fluid handling device by a hanger bar that engages with a hook arm on the fluid handling device so that the shield may be pivoted with respect to the fluid handling device into several positions. It is within the purview of the present invention to include any structure for connecting the shield to the fluid handling device so that the shield may be pivoted with respect to the fluid handling device. These structures include known mechanical hinges and various linkages, living hinges, or combinations of hinges and linkages.

The shield may be connected to the fluid handling device by an interference fit between the hanger bar and the hook arm. Therefore, the shield always is oriented in a stable position and will not move forward or backwards unless movement of the shield relative to the hanger bar and the hook bar is initiated by the user.

Alternatively, the shield and fluid handling device may be a unitary one-piece structure. The one-piece structure may be obtained by many methods, including molding the shield and the fluid handling device as a one-piece unit, thereby eliminating the separate shield and the fluid handling device during the manufacturing assembly process.

The assembly of the present invention may further comprise tactile and visual means for deterring the user from contacting the needle, providing easy orientation of the needle with the patient and providing the user with a guide for actuation and engagement with the shield.

The assembly of the present invention may further comprise means for minimizing exposure by the user to residual fluid leaking from a used needle. For example, a polymer material, such as a gel, may be located in the shield.

Most desirably, the assembly of the present invention is such that the cooperating parts of the assembly provide the means for the shield to move into a forward position over the needle. Thus, by simple movement of the shield into a forward position over the used needle, the assembly is ready for subsequent disposal. Therefore, the safety shield assembly of the present invention provides minimal exposure of the user to a needle because the shielding is initiated by the user immediately after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The fluid handling device of the present invention may comprise a hook arm and the shield may be connected movably to the hook arm. Thus the shield may be position with respect to the fluid handling device and moved easily into several positions.

Preferably, the fluid handling device is fitted non-rotatably with the hub of the needle assembly. Additionally, the fluid handling device includes cooperating means that mate with reciprocal means on the shield to help retain the shield in a final closed position, to propel the shield toward the final closed portion and to provide a clear audible and tactile indication of complete shielding.

The shield preferably includes a cannula finger lock for locked engagement with the cannula when the shield is in the final closed position around the needle cannula. The cannula finger lock preferably projects obliquely from one sidewall of the shield angularly toward the opposed sidewall and the top wall of the shield. The cannula finger lock is dimensioned, disposed and aligned to contact the needle cannula when the shield approaches the final closed position. Contact between the cannula and the cannula finger lock will cause the cannula finger lock to resiliently deflect toward the sidewall from which the cannula finger lock extends. Sufficient rotation of the shield will cause the needle cannula to pass the cannula finger lock. As a result, the cannula finger lock will resiliently return to or toward its undeflected condition for securely trapping the needle cannula in the shield.

Preferably, the fluid handling device is fitted with the hub of the needle assembly so that the fluid handling device cannot rotate around the hub. Additionally, the fluid handling device includes cooperating means that mate with reciprocal means on the shield to propel the shield toward a final closed position.

Alternatively, the fluid handling device and hub may be a unitary one-piece structure. The one piece structure may be accomplished by many methods including molding the fluid handling device and the hub as a one-piece unit thereby eliminating the need to separately assemble the fluid handling device to the hub during the manufacturing process.

Most preferably, the fluid handling device is fitted with the hub of the needle assembly so that the bevel surface or bevel up surface of the intravenous or distal end of the needle faces the same side of the fluid handling device when the shield is in the open position. Alignment of the fluid handling device, hub, shield and needle with the bevel surface up makes it easier to insert the needle into the patient without manipulating the assembly. The orientation of the intravenous end of the needle with the bevel up assures the user that the needle is oriented properly for use and does not require any manipulation before use. Most notably, the orientation of the shield provides a visual indication to the user of the orientation of the bevel surface of the needle.

Alternatively, it is within the purview of the present invention that the shield, fluid handling device and hub is a unitary one-piece structure. The one-piece structure may be accomplished by many methods including molding the shield, fluid handling device and hub as a one piece unit thereby eliminating the need to separately assemble the shield, fluid handling device and hub during the manufacturing process.

Another notable attribute of the present invention is that the tactile and visual features deter the user from touching the needle, allow the user to easily orient the needle with the patient and guide the user to actuate and engage the shield of the assembly.

### BRIEF DESCRIPTION OF THE DRAWINGS

FIG. 1 is an exploded perspective view of a shield, needle holder and needle assembly in accordance with the first embodiment of the invention.

FIG. 2 is an exploded perspective view of a prior art needle assembly for use with the holder of FIG. 1.

FIG. 3 is a longitudinal cross-sectional view showing the shield in the open condition with the needle cannula in the exposed ready-to-use position.

FIG. 4 is a cross-sectional view similar to FIG. 3, but showing the shield in the shielded position.

FIG. 5 is a cross sectional view similar to FIG. 3, but showing a second embodiment with a rotatable flange for releasing the shield.

FIG. 6 is an end elevational view of the embodiments shown in FIG. 5.

FIG. 7 is a perspective view similar to FIG. 1, showing a third embodiment with a rotatable lock for holding the shield in the open condition.

FIG. 8 is a cross-sectional view taken along line 8-8 in FIG. 7.

FIG. 9 is a perspective view similar to FIG. 7, but showing a fourth embodiment of a lock for holding the shield in the open position.

FIG. 10 is a perspective view of a fifth embodiment.

### DETAILED DESCRIPTION OF THE PREFERRED EMBODIMENTS

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

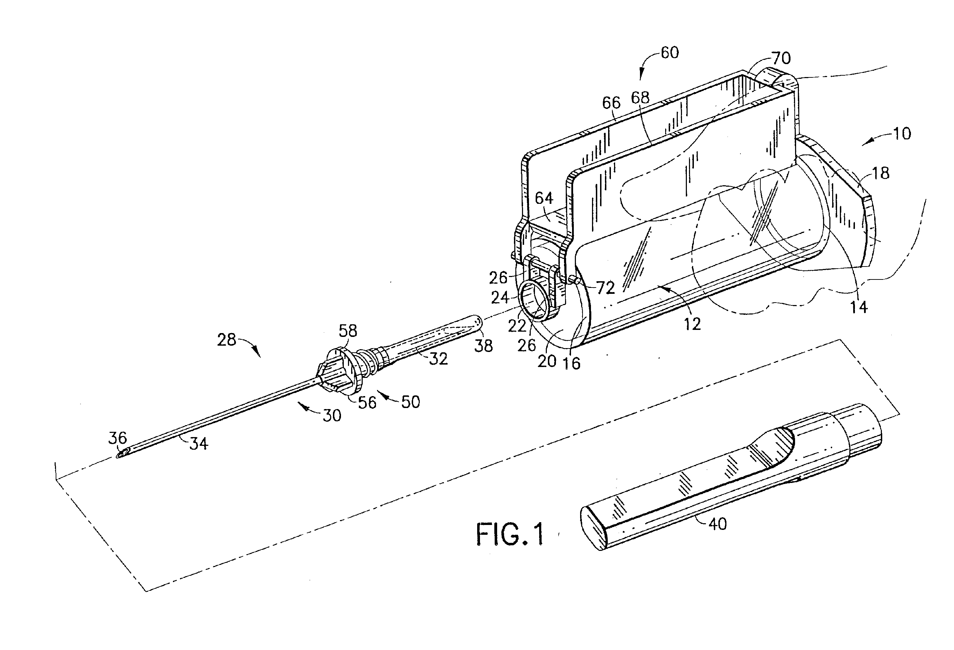

A holder in accordance with the subject invention is identified generally by the numeral **10** in FIG. 1. Holder **10** preferably is formed unitarily from a plastic material and includes a tubular side wall **12** with a proximal end **14** and a distal end **16**. Proximal end **14** of tubular side wall **12** is widely open and is dimensioned to slidably accommodate an evacuated blood collection tube (not shown). A flange **18** projects outwardly at proximal end **14** of tubular side wall **12** to facilitate gripping and manipulation of holder **10.**

An annular distal end wall **20** extends inwardly at distal end **16** of tubular side wall **12**. A central aperture **22** extends through distal end wall **20** and communicates with internal portions of holder **10**. Holder **10** is further characterized by an internally threaded cylindrical collar **24** formed concentrically with aperture **22.** Additionally, hook arms **26** extend distally from distal end wall **20.**

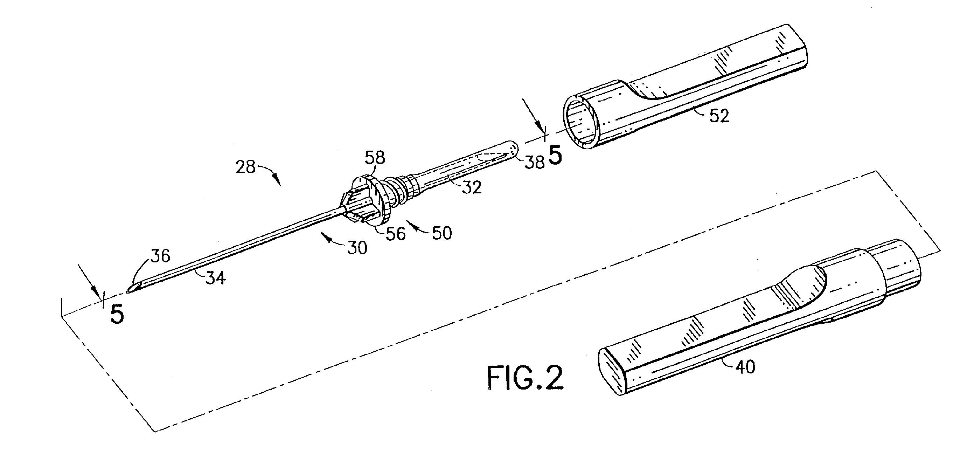

Holder **10** is employed with a known needle assembly **28.** Needle assembly **28** includes a needle **30** with a non-patient end **32,** an intravenous end **34** and a passageway **36** extending between the non-patient end and the intravenous end. An elastomeric sleeve **38** covers the non-patient end. A first rigid sleeve **40** covers the intravenous end and a second rigid sleeve **42** covers the non-patient end and the elastomeric sleeve, as shown in FIG. 2.

Needle assembly **28** further includes a hub **50** with a threaded end **54,** a ribbed end **56** and a passageway extending between the threaded end and the ribbed end. Threaded end **54** and ribbed end **56** are separated by a flange **58.** Non-patient end **32** of needle **30** extends from threaded end **54**, and intravenous end **34** of needle **30** extends from ribbed end **56.** Threaded end **54** comprises male threads for mounting of **50** in internally threaded collar **24** of holder **10**. In the mounted condition, non-patient end **32** of needle **30** projects into the area bounded by cylindrical side wall **12** of holder **10.**

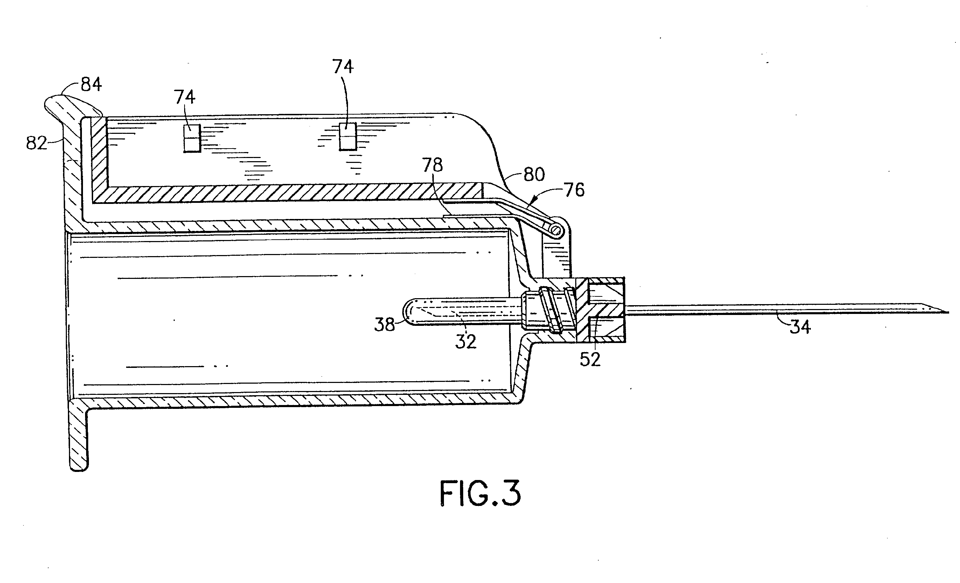

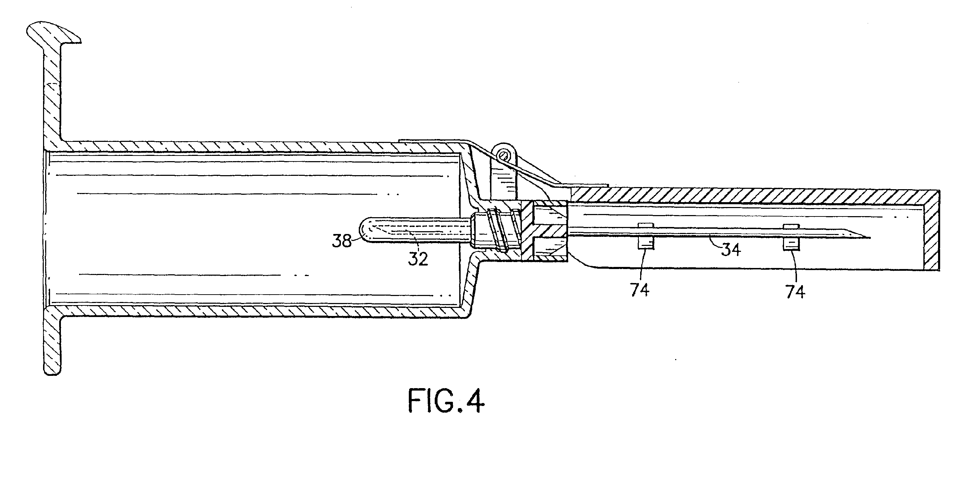

Holder assembly **10** further includes a shield **60** that is formed unitarily from a substantially rigid plastic. Shield **60** includes an elongate base wall **64**, opposed side walls **66** and **68** that project from opposite sides of base wall **64** and an end wall **70** that extends from base wall **64** and between side walls **66** andb Hanger bar **72** projects from an end of shield **60** opposite end wall **70** and is rotatably mounted to hook arms **26**. Thus, shield **60** can be rotated about hook arms **26** from an open position as shown in FIGS. 1 and 3 to a closed position as shown in FIG. 4. Shield **60** is dimensioned such that end wall **70** is substantially adjacent flange **18** of holder **10** when shield **60** is in the open position of FIGS. 1 and 3. Additionally, shield **60** is dimensioned such that end wall **70** is disposed distally of needle **30** when shield **60** is rotated to the closed position of FIG. 4. Side walls **66** and **68** of shield **60** are spaced from one another by a distance that exceeds the width of needle **30.** Furthermore, side walls **66** and **68** and end wall **70** project sufficiently from base wall **64** to completely surround needle **30.**

Shield **60** is formed with needle locks **74** that project from side wall **68** angularly toward base wall **64**. Locks **74** are disposed and configured to contact needle **30** as shield **60** is rotated into the closed position. This contact will cause locks **74** to resiliently deflect. However, locks **74** will return to an undeflected condition and trap needle **30** after shield 60 reaches the closed position of FIG. 4.

Holder assembly **10** further includes a spring **76** mounted to or near hook arms **26.** In the preferred embodiment, spring **76** is a torsion spring having a first leg **78** biased against an external surface of holder **10** and a second leg **80** biased against a surface of shield **60.** Legs **78** and **80** are biased into a stored-energy position when shield **60** is in the open position of FIGS. 1 and 3. However, spring **76** is configured and disposed to bias shield **60** completely to the closed position of FIG. 4. The torsion spring, illustrated in FIGS. 1-4, can be replaced by a leaf spring mounted at substantially the same location on holder **10.** Alternatively, the shield can be joined unitarily to holder **10** by an over-center living hinge. Over-center hinges on a shield are shown, for example, in U.S. Patent No. 5,242,417.

Shield **60** is retained releasably in the open position and against the forces of spring **76** by a latch **82.** Latch **82** protects unitarily from flange **18** and releasably engages an edge of end wall **70** of shield **62** spaced from base wall **64.** Latch **82** is dimensioned and configured to be resiliently deflectable relative to flange **18** and includes an actuator tab **84** dimensioned and configured for convenient digital manipulation.

Holder **10** may be used substantially in a conventional manner to draw samples of blood or other bodily fluid from a patient. In particular, second rigid sleeve **42** is separated from needle assembly **28** to expose elastomeric sleeve **38**. Threaded end **54** of hub **50** then is engaged threadedly in aperture **24** of holder **10**. First rigid sleeve **40** then can be removed from hub **50** to expose intravenous end **34** of needle **30.** Intravenous end **34** of needle **30** then can be placed in communication with a blood vessel or other source of fluid and an evacuated tube can be urged into open proximal end **14** of side wall **12.** Non-patient end **42** of needle **40** thus is placed in communication with the evacuated tube for collecting specimens of blood or other bodily fluid. A plurality of evacuated tubes may be inserted sequentially into holder **10**. After a sufficient volume of fluid has been collected, intravenous end **34** of needle **30** is removed from the patient. The health care worker then deflects latch **82** sufficiently to separate latch **82** from shield **60**. As a result, spring **76** propels shield **60** from the open position shown in FIGS. 1 and 3 to the closed position shown in FIG. 4. Needle locks **74** engage needle **30** as shield **60** is rotated into its closed position.

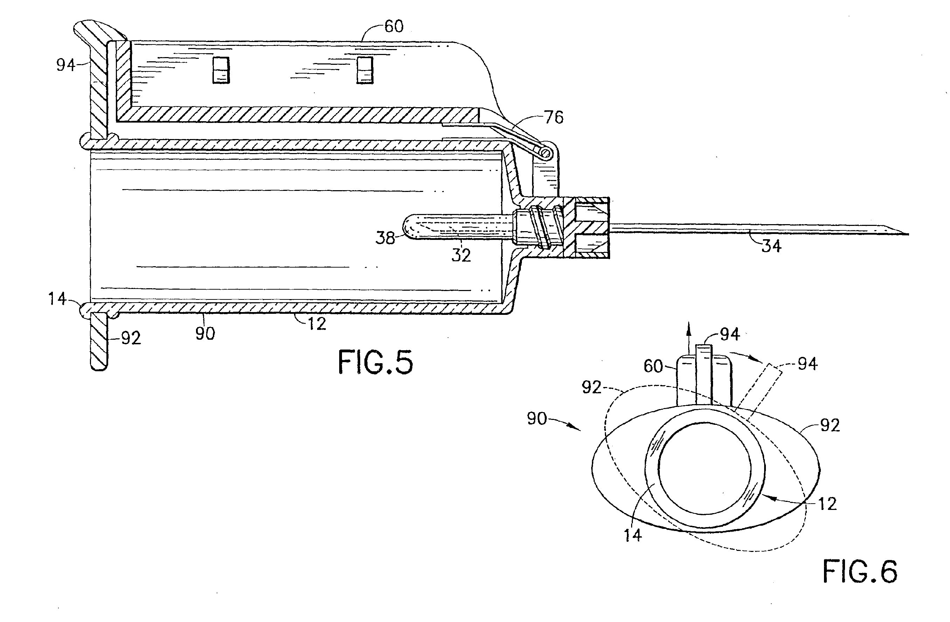

Latch **76** illustrated in FIGS. 1, 3 and 4 is just one of several optional latch configurations. In this regard, a second embodiment of the needle holder **90** is illustrated in FIGS. 5 and 6 and is substantially identical to the first embodiment of FIGS. 1, 3 and 4. However, holder **90** includes a rotatable flange **92**mounted to proximal end **14** of side wall **12.** A latch **94** projects rigidly and substantially non-deflectably from flange **92.** As a result, latch **94** can be separated from shield **60** merely by rotating flange **92** about proximal end **14** of side wall **12** from a first position where latch **94** engages shield **62** to a second position where latch **94** is rotatably spaced from shield **62**, as shown in broken lines in FIG. 6. Thus, shield **60** will be propelled to the closed position by spring **76** after latch **94** is rotated away from shield **60**.

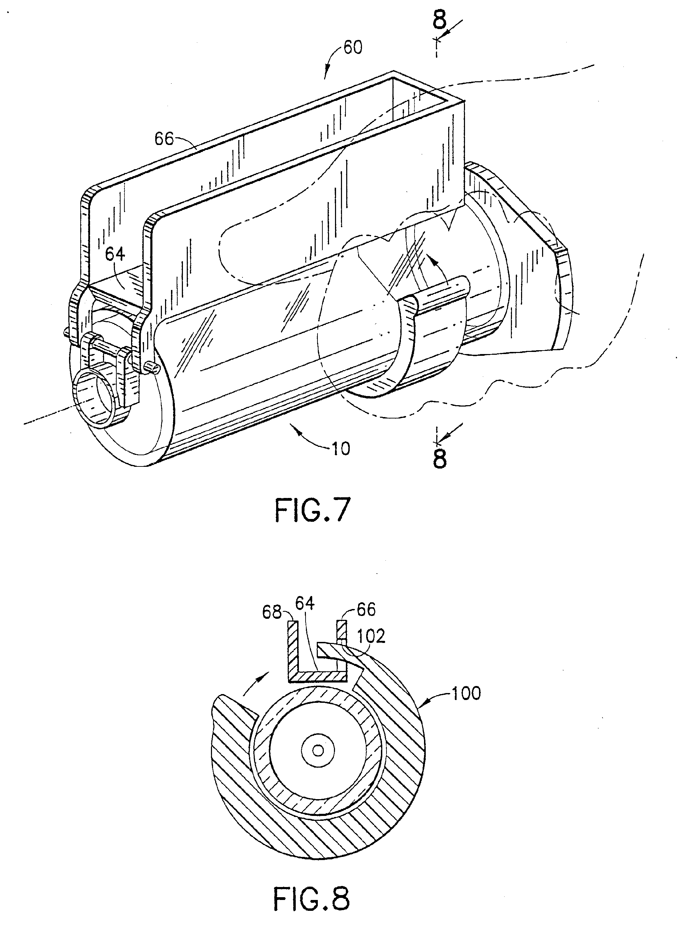

A third embodiment is illustrated in FIGS. 7 and 8, and includes a substantially C-shaped latch **100** rotatably mounted on the exterior of cylindrical side wall **12** of holder **10** at a location adjacent flange **18**. In this embodiment, side wall **66** of shield **60** is formed with a slot **102** adjacent base wall **64** for receiving an end of C-shaped latch **100.** Thus, C-shaped latch **100** can be rotated in the direction of the arrow shown in FIGS. 7 and 8 about the axis of holder **10** from a first position where latch **100** engages in aperture **102** of side wall **68** to a second position where latch **100** is spaced from shield **60**. Thus, shield **60** will be propelled into the closed position when C-shaped latch **100** is spaced from aperture **102** in side wall **68** of shield **60**.

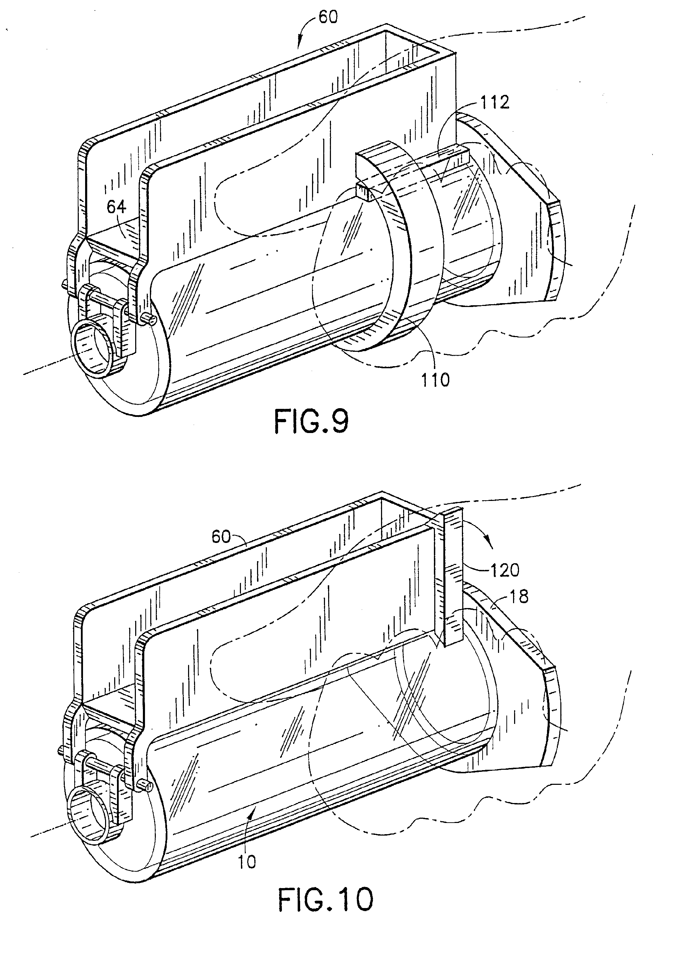

A fourth embodiment is illustrated in FIG. 9 and includes a C-shaped latch **110** that is axially slidable on the exterior of side wall **12** of holder **10**. In a first range of axial movement, latch **110** engages a flange **112** that projects transversely from base wall **64** of shield **60.** Thus, latch **110** prevents shield **60** from being propelled into the closed position. However, a distal movement of latch **110** relative to shield **60,** as shown by the arrow in FIG. 9, separates latch **110** from shield **60** and enables shield **60** to be propelled into the closed position shown in FIG. 4.

A fifth embodiment is illustrated in FIG. 9 and includes a latch **120** that projects transversely outwardly from a portion of tubular side wall **12** of holder **10** adjacent flange **18.** Latch **120**, however, is disposed and configured to be deflected in the directions of the arrow in FIG. 10 about an axis that extends substantially parallel to the axis of holder **10.** In other respects, however, latch **120** is functionally similar to latch **80** described above and illustrated in FIGS. 1, 3 and 4.

While the invention has been described with respect to certain preferred embodiments, it is apparent that variations can be made without departing from the scope of the invention as defined above. For example, the shield can be locked in the closed position by engagement means formed on the shield and on distal portions of the holder or on the needle hub. These locking means on the shield and on the holder or hub can be used in place of cannula latches or in addition to the above-described cannula latches.

## Claims

1. A safety assembly comprising:
a tube holder having a widely open proximal end, a partly closed distal end and a tubular sidewall extending between said ends;
a needle assembly mounted to said distal end of said holder and having a needle cannula with a sharply pointed distal end projecting distally beyond said holder;
a shield hingedly mounted to said proximal end of said holder for movement from a proximal position where said shield is substantially adjacent said tubular sidewall of said holder to a distal position where said shield shields portions of said needle cannula projecting distally from said holder;
a spring disposed for biasing said shield from said proximal position to said distal position; and
a latch disposed on said holder for movement between a locking position where said latch holds said shield in said proximal position and a release position where said latch permits said spring to propel said shield to said distal position.

2. The safety assembly of Claim 1, wherein the shield include at least one lock for securely locking the shield in the distal position.

3. The safety assembly of Claim 2, wherein the lock includes at least one resiliently deflectable cannula locking finger for locked engagement with said needle cannula when said shield is in said distal position.

4. The safety assembly of anyone of the preceding claims, wherein the shield in said proximal position extends substantially from said distal end of said holder to said proximal end thereof.

5. The safety assembly of anyone of the preceding claims, wherein said distal position of said shield is spaced from said proximal position of said shield by approximately 180°.

6. The safety assembly of anyone of the preceding claims, wherein said shield defines a substantially U-shaped channel having an open side facing away from said holder when said shield is in said proximal position.

7. The safety assembly of anyone of the preceding claims, wherein said latch extends integrally from said proximal end of said holder and is deflectable about an axis extending substantially parallel to an axis defined by the hinged connection of said shield to said holder.

8. The safety assembly anyone of Claims 1 to 6, wherein said latch extends integrally from said proximal end of said holder and is deflectable about an axis extending substantially orthogonal to an axis defined by the hinged connection of said shield to said holder.

9. The safety assembly of anyone of the preceding claims, wherein said latch is rotatably mounted to said holder for rotation about an axis substantially parallel to an axis defined by said tubular sidewall of said holder.

10. The safety assembly of anyone of the preceding claims, wherein said latch is movable along said holder substantially parallel to an axis defined by said tubular sidewall of said holder.
